# EUROPEAN PATENT APPLICATION

(11) **EP 4 834 916 A1**
(43) Date of publication of application: **04.11.2026**
(21) Application number: 24910242.7
(22) Date of filing: 25.10.2024
(51) Int. Cl.: C07K 7/08, C07K 14/00, C12N 15/11, A61K 38/10, A61P 9/10

(54) **PHARMACEUTICALLY ACCEPTABLE SALT OF POLYPEPTIDE AND USE THEREOF**

(30) Priority: 27.12.2023 CN 202311825930
(71) Applicant: Biocells (Beijing) Biotech Co., Ltd., Beijing 100070 (CN)
(72) Inventor: HAN, Huamin, Beijing 100070 (CN); JI, Qi, Beijing 100070 (CN)
(74) Representative: Mewburn Ellis LLP
(86) International application number: PCT/CN2024/127363
(87) International publication number: WO 2025/139301

(57) **Abstract**

A pharmaceutically acceptable salt of a polypeptide and a use thereof. According to the pharmaceutically acceptable salt of the polypeptide, the half-life of a drug is prolonged by means of a study on the structure-activity relationship and structural modification, the in-vivo exposure is increased, the number of times of administration is reduced, the side effect in animal experiments is lower, and the therapeutical window of the drug is larger. The pharmaceutically acceptable salt of the polypeptide can be used for treatment of ETBR-related diseases, such as rehabilitation treatment of the acute phase and the recovery phase of a cerebral arterial thrombosis patient, promoting better recovery of the stroke patient.

## Description

### TECHNICAL FIELD

The present invention relates to the field of biotechnology, and particularly to a pharmaceutically acceptable salt of a polypeptide and use thereof.

### BACKGROUND

Current therapeutic measures and medications for stroke primarily include vascular intervention, thrombolysis, antiplatelet therapy, anticoagulant therapy, defibrinogenating therapy, and neuroprotective therapy. Generally, vascular intervention and thrombolysis are required to be performed at the ultra-early stage after stroke onset, and vascular intervention needs to be implemented in a hospital with certain capabilities. Antiplatelet drugs and anticoagulant drugs are generally used in combination with thrombolytic drugs in an advanced stage, with limited efficacy and a certain risk of bleeding. Although neuroprotectants possess promising potential, most are generally used for acute stroke treatment. Currently, there is still no marketed therapeutic drug for promoting rehabilitation in stroke patients during the subacute and recovery phase.

Previous studies have shown that endothelin B receptor agonists exhibit prominent potential in promoting long-term recovery and late rehabilitation of stroke patients. Endothelins (ETs) are polypeptides containing 21 amino acids, which can be divided into three subtypes: ET-1, ET-2 and ET-3. These subtypes share high structural homology with snake venom sarafotoxins b and c (S6b, S6c). Endothelins are known as the most potent and long-acting vasoactive peptides at present, and have strong vasoconstrictive effects and can promote the migration and proliferation of smooth muscle cells. They exert extensive effects on various systems in the body and are closely associated with diseases such as hypertension, congestive heart failure, diabetes, cancer and fibrosis.

Endothelins exert their biological effects mainly via binding to membrane-bound endothelin receptors (ETRs) on target cells, and play an important role in the occurrence and progression of various diseases, including cardiovascular and cerebrovascular diseases, kidney diseases, diabetes, autoimmune diseases and tumors. Human ETRs are divided into two subtypes: ETA and ETB, both of which are G-protein-coupled receptors (GPCRs), but have different functions. ETA is considered as a primary vasoconstrictive and growth-promoting receptor, while ETB inhibits cell growth and vasoconstriction in the vascular system and is called a "clearance receptor" as it is involved in the clearance of ET-1. Endothelins and their receptors are diversely and widely distributed in various tissues, mediate complex biological effects through different subtypes and signaling systems, and are closely associated with a variety of diseases, where ETA and ETB receptors play important roles.

Studies have shown that endothelin B receptor (ETBR) agonists exhibit prominent potential in promoting the regeneration of neurovascular units, improving the blood supply and oxygen supply to the nervous system, promoting the long-term recovery and late rehabilitation of stroke patients. Among the currently discovered ETBR agonist compounds, sovateltide (IRL-1620, Suc-DEEAVYFAHLDIIW) is the only ETBR agonist that has been advanced to the clinical research stage. However, IRL-1620 has a very short half-life (less than 2 min) and a very low exposure. Repeated administrations are required in preclinical studies and clinical studies, which greatly reduces its duration of efficacy and patient compliance.

Accordingly, there is an unmet urgent need to develop novel endothelin receptor agonists.

### SUMMARY

In view of the above, the present invention provides a novel pharmaceutically acceptable salt of a polypeptide and use thereof.

The polypeptide and the pharmaceutically acceptable salts thereof provided in the present invention are developed on the basis of mechanistic studies of ETBR agonists, and are highly selective agonists of ETBR. Through structure-activity relationship studies and structural modification, the polypeptide and the pharmaceutically acceptable salts thereof provided in the present invention prolong the half-life, increase *in-vivo* exposure, reduce the dosing frequency, exhibit lower side effects in animal experiments than IRL-1620, the only ETBR agonist that has entered the clinical research stage, and possess a larger safety window. The polypeptide and a pharmaceutically acceptable salt thereof can be used for the treatment of ETBR-related diseases, such as rehabilitation during the recovery period of patients with ischemic stroke, thereby promoting better recovery.

Specifically, the present invention provides the following technical solutions.

In a first aspect, the present application provides a pharmaceutically acceptable salt of a polypeptide, wherein the polypeptide comprises the amino acid sequence Fum-DEEAVYFAHK(-AEEA-γE-C12)DVIW (SEQ ID NO: 1) or functional variants thereof.

In some embodiments, the functional variants are those resulting from one or more conservative substitutions in SEQ ID NO: 1.

In some embodiments, the conservative substitution is selected from the group consisting of substitutions between D and E, among V, L and I, among Y, F and W, and among H, K and R in SEQ ID NO: 1.

In some embodiments, the functional variants have the same or similar ETBR agonistic activity as the polypeptide of SEQ ID NO: 1.

In some embodiments, the pharmaceutically acceptable salt of the polypeptide is selected from the group consisting of a sodium salt, a potassium salt, an ammonium salt, trifluoroacetate, acetate, hydrochloride, sulfate, and phosphate.

In some embodiments, the pharmaceutically acceptable salt of the polypeptide is selected from the group consisting of a sodium salt, a potassium salt, and an ammonium salt.

In some embodiments, the pharmaceutically acceptable salt of the polypeptide is a sodium salt.

In a second aspect, the present invention provides a pharmaceutical composition comprising the pharmaceutically acceptable salt of the polypeptide of the first aspect, and a pharmaceutically acceptable carrier, excipient, and/or diluent.

In some embodiments, the pharmaceutical composition is a pre-lyophilized formulation.

In some embodiments, the pharmaceutical composition comprises sodium chloride and/or optional trehalose and/or cyclodextrin.

In some embodiments, the pharmaceutical composition is a lyophilized formulation, preferably prepared by lyophilizing the pre-lyophilized formulation described above.

In some embodiments, the pharmaceutical composition is a reconstituted preparation, preferably prepared by reconstituting the lyophilized preparation described above with an aqueous solution.

In some embodiments, the pharmaceutical composition can be used to treat ETBR-related diseases.

In some embodiments, the pharmaceutical composition can be used to treat, ameliorate, or prevent neurological injury and secondary disorders resulting therefrom, neurodegenerative diseases, anxiety disorders, epileptic disorders, aortic stenosis, or neonatal hypoxic-ischemic encephalopathy.

In a third aspect, the present invention provides a method for promoting the regeneration of neurovascular units, improving the blood supply and oxygen supply to the nervous system, or treating, ameliorating or preventing neurological injury and secondary disorders resulting therefrom, neurodegenerative diseases, anxiety disorders, epileptic disorders, aortic stenosis, or neonatal hypoxic-ischemic encephalopathy in a subject, comprising administering to the subject the pharmaceutically acceptable salt of the polypeptide of the first aspect, or the pharmaceutical composition of the second aspect.

In a fourth aspect, the present application provides use of the pharmaceutically acceptable salt of the polypeptide of the first aspect or the pharmaceutical composition of the second aspect in the manufacture of a medicament for promoting the regeneration of neurovascular units, improving the blood supply and oxygen supply to the nervous system, or treating, ameliorating or preventing neurological injury and secondary disorders resulting therefrom, neurodegenerative diseases, anxiety disorders, epileptic disorders, aortic stenosis, or neonatal hypoxic-ischemic encephalopathy in a subject.

In some embodiments of the second, third, or fourth aspect, the neurological injury and secondary disorders resulting therefrom are selected from the group consisting of stroke, spinal cord injury, ischemic or traumatic brain or spinal cord injury, and central nervous system (CNS) neuronal injury.

In some embodiments of the second, third, or fourth aspect, the stroke is selected from ischemic stroke, hemorrhagic stroke, and hemorrhagic stroke converted from ischemic stroke.

In some embodiments of the second, third, or fourth aspect, the neurodegenerative diseases are selected from the group consisting of Alzheimer's disease, amyotrophic lateral sclerosis (ALS), Parkinson's disease, and Huntington's disease.

In some embodiments of the second, third or fourth aspect, the subject is a mammal, such as a non-primate or a primate, such as a human.

In a fifth aspect, the present invention provides a medicament comprising the pharmaceutically acceptable salt of the polypeptide of the first aspect or the pharmaceutical composition of the second aspect, and optional any other active ingredient.

The present invention further provides a method of preparing the pharmaceutically acceptable salt of the polypeptide, comprising the following steps:
Step 1: obtaining a peptide resin of the polypeptide via solid-phase polypeptide synthesis;
Step 2: cleaving and purifying to obtain the polypeptide; and
Step 3: preparing the salt from the polypeptide.

In addition, the present invention further provides use of the polypeptide (SEQ ID NO: 1) and a pharmaceutically acceptable salt thereof in the manufacture of a medicament for the treatment of ETBR-related diseases in a subject.

In some embodiments, the ETBR-related diseases comprise one or more selected from the group consisting of neurological injury and secondary disorders resulting therefrom, neurodegenerative diseases, anxiety disorders, epileptic disorders, aortic stenosis, and neonatal hypoxic-ischemic encephalopathy.

In some embodiments, the neurological injury and secondary disorders resulting therefrom are selected from the group consisting of stroke, spinal cord injury, ischemic or traumatic brain or spinal cord injury, and central nervous system (CNS) neuronal injury.

In some embodiments, the neurodegenerative diseases are selected from the group consisting of Alzheimer's disease, amyotrophic lateral sclerosis (ALS), Parkinson's disease, and Huntington's disease.

In some embodiments, the stroke is selected from the group consisting of ischemic stroke, hemorrhagic stroke, and hemorrhagic stroke converted from ischemic stroke.

Accordingly, the present invention further provides a medicament comprising the pharmaceutically acceptable salt of the polypeptide, and a pharmaceutically acceptable additive or auxiliary.

In some embodiments, the medicament further comprises any other active ingredient.

The present application further provides a method for treating ETBR-related diseases, comprising administering to a subject a pharmaceutically acceptable salt of the polypeptide, the pharmaceutical composition, or the medicament.

In some embodiments of the present invention, the ETBR-related diseases comprise one or more selected from the group consisting of neurological injury and secondary disorders resulting therefrom, neurodegenerative diseases, anxiety disorders, epileptic disorders, aortic stenosis, and neonatal hypoxic-ischemic encephalopathy.

In some embodiments, the neurological injury and secondary disorders resulting therefrom are selected from the group consisting of stroke, spinal cord injury, ischemic or traumatic brain or spinal cord injury, and central nervous system (CNS) neuronal injury.

In some embodiments, the neurodegenerative diseases are selected from the group consisting of Alzheimer's disease, amyotrophic lateral sclerosis (ALS), Parkinson's disease, and Huntington's disease.

In some embodiments, the stroke is selected from the group consisting of ischemic stroke, hemorrhagic stroke, and hemorrhagic stroke converted from ischemic stroke.

### BRIEF DESCRIPTION OF THE DRAWINGS

In order to describe the technical solutions of the embodiments of the present invention or the prior art more clearly, a brief description of the accompanying drawings required for use in the description of the embodiments or the prior art will be made below.
FIG. 1 shows the results of EC50 determination for the activity of the polypeptides toward the human ETB receptor in Example 2.
FIG. 2 shows the results of EC50 determination for the activity of the polypeptides toward the canine ETB receptor in Example 2.
FIG. 3 shows the results of EC50 determination for the activity of the polypeptides toward the rat ETB receptor in Example 2.
FIG. 4 shows the results of EC50 determination for the activity of the polypeptides toward the mouse ETB receptor in Example 2.
FIG. 5 shows the Garcia JH score results on day 7 following administration three times in one week in Example 9, where group 1 is the normal saline group, groups 2 and 3 are subcutaneous administration of 30 µg/kg and 100 µg/kg BX-229-Na, respectively, and group 4 is the sham group.
FIG. 6 shows the Garcia JH score results on day 14 following administration three times in one week in Example 9, where group 1 is the normal saline group, groups 2 and 3 are subcutaneous administration of 30 µg/kg and 100 µg/kg BX-229-Na, respectively, and group 4 is the sham group.
FIG. 7 shows the changes in VEGF-A protein expression following administration three times in one week in Example 9, where group 1 is the normal saline group, groups 2 and 3 are the subcutaneous administration of 30 µg/kg and 100 µg/kg BX-229-Na, respectively, and group 4 is the sham group.
FIG. 8 shows the changes in BDNF protein expression following administration three times in one week in Example 9, where group 1 is the normal saline group, groups 2 and 3 are the subcutaneous administration of 30 µg/kg and 100 µg/kg BX-229-Na, respectively, and group 4 is the sham group.
FIG. 9 shows the score results on days 8 and 15 of the BX-229 subcutaneous administration group and the IRL-1620 intravenous administration group after modeling in Example 10 (* represents significant difference, P < 0.05), where groups 1 and 3 are subcutaneous administration of 30 µg/kg BX-229-Na, and groups 2 and 4 are intravenous administration of 3×2.7 µg/kg IRL-1620.

### DETAILED DESCRIPTION

The present invention discloses a pharmaceutically acceptable salt of a polypeptide and use thereof. With reference to the content herein, those skilled in the art may implement the present invention by appropriately modifying process parameters. Particularly, it should be noted that all similar alternatives and modifications will be apparent to those skilled in the art, and all of them are considered to be included in the present invention. The methods and use of the present invention have been described in terms of preferred Examples. Apparently, those skilled in the art can make modifications or appropriate changes and combinations to the methods and use described herein without departing from the content, spirit, and scope of the present invention to implement and use the technology of the present invention.

Unless otherwise indicated, the terms used in this application have the meanings commonly understood by those skilled in the art.

Single-letter or three-letter abbreviations used for amino acids in the present invention follow international practice.

In this specification and claims, the words "including", "comprising" and "containing" mean "including but not limited to" and are not intended to exclude other parts, additives, components or steps.

In a first aspect, the present invention provides a pharmaceutically acceptable salt of a polypeptide, wherein the polypeptide comprises the amino acid sequence Fum-DEEAVYFAHK(-AEEA-γE-C12)DVIW(SEQ ID NO: 1) or functional variants thereof.

The term "functional variants" refer to those having the same or similar biological functions and properties as the parent. As a non-limiting example, "functional variants" can be obtained by performing one or more conservative substitutions in the parent.

In some embodiments, the conservative substitution is selected from the group consisting of substitutions between D and E, among V, L and I, among Y, F and W, or among H, K and R.

In some embodiments, the functional variants disclosed herein also include amino acid sequences that have at least 60%, 65%, 70%, 75%, 80%, 85%, 90%, 91%, 92%, 93%, 94%, 95%, 96%, 97%, 98%, 99% or more identity to the above-mentioned polypeptide. It is known in the art that "identity" between two proteins/polypeptides is determined by aligning the amino acid sequence of one protein/polypeptide with the sequence of second protein/polypeptide containing conservative amino acid substitutions thereof. The degree of identity between the two proteins/polypeptides is determined using computer algorithms and methods well known to those skilled in the art. The identity between the two amino acid sequences is preferably determined by using the BLASTP algorithm.

In some embodiments, the functional variants disclosed herein include those that differ from the above-mentioned polypeptide by having substitutions, deletions, additions, and/or insertions of 1, 2, 3, 4, 5, or more amino acid residues.

As described above, the functional variants may differ from the above - mentioned polypeptide by one or more substitutions, deletions, additions, and/or insertions. These variants may be naturally occurring or may be synthetically produced, for example, by modifying one or more of the above-mentioned polypeptide sequences disclosed herein and assessing their biological activity as described herein using any of techniques well known in the art.

The active polypeptide of the present invention can be synthesized by solid phase synthesis or recombinant methods. Peptidomimetics can be synthesized by various schemes and methods described in the scientific and patent literature, for example, Organic Syntheses Collective Volumes, Gilman et al.(ed.), John Wiley&Sons, Inc., NY, al-Obeidi (1998) Mol. Biotechnol. 9: 205-223;Hruby (1997) Curr. Opin. Chem. Biol. 1:114-119;Ostergaard (1997) Mol. Divers. 3: 17-27;Ostresh (1996) Methods Enzymol. 267: 220-234.

In addition, the present invention provides a method of preparing a pharmaceutically acceptable salt of polypeptide (SEQ ID NO: 1), comprising the following steps.

Step 1. 0.50 g (0.25 mmol) Fmoc-Trp(Boc)-Wang Resin is weighed and added into a reactor. 10 mL DCM was added for resin swelling for 10 min, filtered by suction, and the resin was washed twice with DMF. 25% 4-methylpiperidine/DMF (v/v) is added and reacted for 30 min to remove the Fmoc protecting group. After suction filtration, the resin is washed four times with DMF and twice with DCM. A ninhydrin test is performed, and the resin solution is found to be blue. Fmoc-Ile-OH (1 mmol, 4 eq.) and HBTU (1 mmol, 4 eq.) are weighed, dissolved in DMF, added with DIEA (2 mmol, 8 eq.), and mixed homogeneously. The mixture is added to the resin and incubated for 1 h under magnetic stirring at room temperature (25 °C ± 5 °C). After suction filtration, the resin is washed sequentially with DMF, MeOH, and DCM, three times for each solvent. A ninhydrin test is performed, and the resin is found to be colorless and transparent, while the solution is found to be light yellow, indicating a complete reaction. 25% 4-methylpiperidine/DMF (v/v) is added to remove the Fmoc protecting group. This deprotection-coupling cycle is repeated to complete sequential coupling of Val, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF is added to the peptide resin and reacted for 10 min for 3 cycles to remove the side-chain Dde protecting group of Lys. AEEA, Glu-α-OtBu, and C12-OH are then condensed sequentially.

Step 2. The peptide resin is dried by suction, and 15 mL pre-cooled cleavage solution (TFA: TIS: H₂O = 95:2.5:2.5) is added. The cleavage reaction is carried out for 3 h at room temperature (25 °C ± 5 °C) with continuous stirring. The reaction solutions are extracted by suction, and the resin is washed twice with a small amount of TFA followed by suction. The reaction solutions are combined, and approximately 200 mL pre-cooled methyl tert-butyl ether is added to precipitate white solids. After centrifugation (5000 rpm for 5 min), the supernatant is discarded. Methyl tert-butyl ether is added again to the precipitate again, followed by vortexing and centrifugation, and the supernatant is discarded. The precipitate is dried in a vacuum dryer for 12 h to obtain crude peptide. The crude peptide is dissolved in approximately 1% aqueous ammonia solution and filtered. The filtrate is purified via preparative liquid phases. Preparative column: C18-10-100, 30×250 mm. Flow rate: 25 mL/min. Phase A: 0.1% TFA/water, and Phase B: 0.1% TFA/90% acetonitrile/water. Gradient elution is performed to collect target fractions. Fractions with purity higher than 95% are combined, and acetonitrile is removed under reduced pressure at around 40 °C via a water pump-equipped rotary evaporator. The concentrated solution is frozen via liquid nitrogen and lyophilized for 48 h in a freeze-dryer to obtain the target compound. The molecular weight was confirmed correct by mass spectrometry ([M-2]²⁻=1137.3).

### Step 3: Preparation of Sodium salt of polypeptide (SEQ ID NO: 1) (BX-229-Na)

The preparation of the sodium salt is exemplified herein.

The polypeptide (SEQ ID NO: 1) solid obtained in step 2 is dissolved in 0.1 N aqueous sodium hydroxide solution (containing 10 equivalents of NaOH), and salt conversion is performed via preparative liquid chromatography. Preparative column: C18-10-100, 30×250 mm. Flow rate: 25 mL/min. Phase A: water, and Phase B: acetonitrile. After sample loading, the column is rinsed with pure water for 15 min, and then the target product is eluted by gradient elution. Fractions with purity higher than 95% are combined, and acetonitrile is removed under reduced pressure at around 40 °C via a water pump-equipped rotary evaporator. The concentrated solution via liquid nitrogen and lyophilized for 48 h in a freeze-dryer to obtain the final sodium salt product BX-229-Na.

In some specific embodiments of the present invention, the preparative column used in the purification includes C18-10-100, 30×250 mm, and the flow rate is 25 mL/min; the preparative liquid phases include Phase A: 0.1% TFA/water, and Phase B: 0.1% TFA/90% acetonitrile/water.

Without wishing to be bound by any theory, it is expected that certain undesirable physicochemical or biopharmaceutical properties of a drug can be improved by combining a molecule or ion having a charge opposite to that of the drug with the drug to form a salt, such as altering the solubility or dissolution of the drug, reducing hygroscopicity of the drug, increasing stability of the drug, and altering the melting point of the drug. The final determination of the desired salt form requires finding a balance between physicochemical and biopharmaceutical properties. The following requirements should be given priority in selecting a pharmaceutically acceptable salt form of a drug: solubility, hygroscopicity, stability to environmental factors in different states.

The pharmaceutically acceptable salt of the polypeptide of the present invention can be in any suitable pharmaceutically acceptable salt form. In some embodiments, the pharmaceutically acceptable salt of the polypeptide is a sodium salt. In some embodiments, the pharmaceutically acceptable salt of the polypeptide is a potassium salt. In some embodiments, the pharmaceutically acceptable salt of the polypeptide is an ammonium salt. In some embodiments, the pharmaceutically acceptable salt of the polypeptide is trifluoroacetate or acetate. In some embodiments, the pharmaceutically acceptable salt of the polypeptide is hydrochloride, sulfate, or phosphate.

In a second aspect, the present application provides a pharmaceutical composition comprising the pharmaceutically acceptable salt of the polypeptide of the first aspect, and a pharmaceutically acceptable carrier, excipient, and/or diluent.

The pharmaceutically acceptable salt of the polypeptide described in the present invention can be prepared as a lyophilized formulation. In some embodiments, the present invention provides a lyophilized formulation. The lyophilized formulation is prepared by lyophilizing a pre-lyophilized formulation, comprising at least an active ingredient, a buffer, a filler, and water, wherein the active ingredient is the pharmaceutically acceptable salt of the polypeptide of the present invention. The filler provides structure for the lyophilized product. In some embodiments, the filler is selected from the group consisting of mannitol, trehalose, dextran-40, glycine, lactose, sorbitol, cyclodextrin, and sucrose, and the like, with trehalose and cyclodextrin being preferred. In some embodiments, the lyophilized formulation of the present invention comprises the pharmaceutically acceptable salt of the polypeptide described above, as well as trehalose and cyclodextrin.

The lyophilized formulation can be reconstituted, i.e., rehydrated with a solution to form a solution free of visible microparticles. In some embodiments, the present invention provides a reconstituted formulation prepared by reconstituting the lyophilized formulation with an aqueous solution. In some embodiments, the aqueous solution is water for injection. In some embodiments, the aqueous solution is normal saline.

The term "lyophilization" refers to a process in which raw materials to be dried are first frozen and then sublimed under a vacuum to remove ice or frozen solvents.

In some embodiments, the pharmaceutically acceptable salt of the polypeptide disclosed herein can be administered in the form of a pharmaceutical composition. The pharmaceutical composition can be prepared by conventional mixing, dissolving, granulating, tableting, grinding, emulsifying, encapsulating, entrapping, or lyophilization techniques.

The pharmaceutical compositions may be formulated in a conventional manner using one or more physiologically acceptable carriers, diluents, excipients or adjuvants that facilitate processing of the pharmaceutically acceptable salt of the polypeptide into pharmaceutical preparations. Proper formulation depends on the chosen route of administration.

In some embodiments, the administration route can be parenteral, intravenous, oral, subcutaneous, intraarterial, intracranial, intrathecal, intraperitoneal, topical, intranasal, or intramuscular, with intravenous administration being preferred.

In some embodiments, the pharmaceutical composition for parenteral administration is preferably sterile and substantially isotonic. For injection, the pharmaceutically acceptable salt of the polypeptide may be formulated into an aqueous solution, preferably in physiologically compatible buffers such as Hank's solution, Ringer's solution, physiological saline, or acetate buffer to alleviate irritation at the injection site. Such Solutions may contain suspending, stabilizing, and/or dispersing agents.

Alternatively, the pharmaceutically acceptable salt of the polypeptide may be in powder form for reconstitution with a suitable carrier (e.g., sterile pyrogen-free water) prior to use.

For transmucosal administration, penetration enhancers suitable for the biological barrier to be penetrated are incorporated into the formulation. This administration route can be used to deliver the compound to the nasal cavity or for sublingual delivery.

In some embodiments, for oral administration, the pharmaceutically acceptable salt of the polypeptide can be formulated together with pharmaceutical carriers into tablets, pills, lozenges, capsules, liquids, gels, syrups, slurries, suspensions, and the like, for oral ingestion by the patients being treated. For oral solid dosage forms such as powders, capsules and tablets, suitable excipients include fillers, for example sugars such as lactose, sucrose, mannitol, and sorbitol; cellulose-based materials, for example, corn starch, wheat starch, rice starch, potato starch, gelatin, tragacanth, methylcellulose, hydroxypropylmethylcellulose, sodium carboxymethylcellulose, and/or povidone (PVP); granulating agents and binders. If desired, disintegrants such as cross-linked polyvinylpyrrolidone, agar, alginic acid or a salt thereof (e.g., sodium alginate), may be added. If desired, solid dosage forms can be sugar-coated or enteric-coated using conventional manufacturing techniques. For oral liquid preparations such as suspensions, elixirs and solutions, suitable carriers, excipients or diluents include water, glycerin, vegetable oils, and alcohols. Flavoring agents, preservatives, colorants, and the like may additionally be added.

In addition to the aforementioned formulations, the pharmaceutically acceptable salt of the polypeptide can also be formulated as depot preparations. Such long-acting formulations can be administered via implantation (e.g., subcutaneous or intramuscular) or intramuscular injection. Therefore, for example, the compound can be formulated together with a suitable polymeric or hydrophobic material (e.g., as a pharmaceutically acceptable oil emulsion), an ion exchange resin, or a sparingly soluble derivative, such as a sparingly soluble salt.

Alternatively, other drug delivery systems may be utilized. Liposomes and emulsions maybe used to deliver the pharmaceutically acceptable salt of the polypeptide. Certain organic solvents such as dimethyl sulfoxide can also be used. Additionally, the pharmaceutically acceptable salt of the polypeptide can be delivered via sustained-release systems, such as semipermeable matrices formed from solid polymers loaded with the therapeutic agent.

Depending on its chemical properties, a sustained-release capsule can release the pharmaceutically acceptable salt of the polypeptide over a period ranging from several weeks to in excess of 100 days. Additional protein stabilization strategies may be selected based on the chemical properties and biological stability of the therapeutic agent.

The pharmaceutically acceptable salt of the polypeptide is administrated in a therapeutically effective amount to achieve the intended therapeutic objective (e.g., alleviating tissue injury resulting from traumatic stroke and related conditions). A therapeutically effective amount refers to an amount of the pharmaceutically acceptable salt of the polypeptide sufficient to significantly reduce stroke-induced injury in the patients (or animal models) being treated, compared with central nervous system injury observed in the untreated control patients (or animal models). An amount is also considered therapeutically effective if an individual patient being treated achieves superior clinical outcomes, as determined by infarct volume or disability scale scores, compared with the mean outcomes observed in an untreated control patient cohort. An amount is also considered to be a therapeutically effective amount if an individual patient being treated exhibits a modified Rankin Scale score of 2 or lower, and a Barthel Index score of 75 or higher. If the patient population being treated exhibits a significantly superior disability scare score distribution (i.e., reduced disability severity) compared with a matched untreated population, the dose is also considered therapeutically effective, see Lees et al., N Engl J Med 2006; 354:588-600. A therapeutically effective regimen means the combination of a therapeutically effective dose and administration frequency necessary to achieve the above-mentioned therapeutic objectives.

In some embodiments, the amount of pharmaceutically acceptable salt of the polypeptide administered depends on the subject being treated, the weight of the subject, disease severity, administration route, and the clinical adjustment of the prescribing physician. Treatment cycles may be repeated when symptoms are detectable, or even undetectable. Treatment may be administrated as monotherapy or in combination with one or more additional therapeutic agents.

In some embodiments, a therapeutically effective amount of the pharmaceutically acceptable salt of the polypeptide disclosed herein can provide therapeutic benefits without inducing significant toxic effects. Toxicity profiles of the pharmaceutically acceptable salt of the polypeptide may be determined in cell culture systems or laboratory animals using standard preclinical pharmacology protocols, for example, via determination of the LD50 (the dose lethal to 50% of the test population) or LD100 (the dose lethal to 100% of the test population). The ratio between the toxic dose and the therapeutically effective dose is defined as the therapeutic index. The pharmaceutically acceptable salts of the polypeptide exhibiting a high therapeutic index are preferred (see, e.g., Fing et al., 1975, In: The Pharmacological Basis of Therapeutics, Chapter 1, page 1).

In a third aspect, provided in the present application is a method of treating, ameliorating, or preventing ETBR-related diseases in a subject.

In some embodiments, the ETBR-related diseases include one or more of neurological injury and secondary disorders resulting therefrom, neurodegenerative diseases, anxiety disorders, epileptic disorders, aortic stenosis, and neonatal hypoxic-ischemic encephalopathy; and the neurological injury and secondary disorders resulting therefrom include stroke, spinal cord injury, ischemic or traumatic brain or spinal cord injury, and central nervous system (CNS) neuronal injury.

In some embodiments, the neurodegenerative diseases include Alzheimer's disease, amyotrophic lateral sclerosis (ALS), Parkinson's disease, and Huntington's disease;
In some embodiments, the stroke includes ischemic stroke, hemorrhagic stroke, and hemorrhagic stroke converted from ischemic stroke.

In some embodiments, the method comprises administering to a subject the pharmaceutically acceptable salt of the polypeptide of the first aspect, or the pharmaceutical composition of the second aspect.

Stroke is a condition caused by impaired blood flow in the CNS. Possible causes include embolism, bleeding, and thrombosis. The death of CNS tissue is called infarction. Infarct volume (i.e., the volume of dead neuronal cells in the brain caused by stroke) can be used as an indicator of the degree of pathological damage caused by stroke. Symptomatic effects depend on both the infarct volume and the location of the infarct in the brain. Disability index can be used as a measure of symptomatic injury, e.g., Rankin Stroke Outcome Scale (Rankin, Scott Med J;2: 200-15 (1957)) and Barthel index. The Rankin scale is based on a direct evaluation of the patient's global condition as follows.
0-No symptoms at all.
1-No significant disability despite symptoms; able to carry out all daily work and activities.
2-Slight disability; unable to perform all previous activities, but able to look after their own affairs without assistance.
3-Moderate disability requiring some help, but able to walk without assistance.
4-Moderate to severe disability; unable to walk without assistance and unable to attend to their own bodily needs without assistance.
5-Severe disability; bedridden, incontinent, and requiring constant nursing care and attention.

The Barthel index is based on a series of questions regarding a patient's ability to perform 10 basic activities of daily living, with scores ranging between 0 and 100, where a lower score indicates more disability (Mahoney et al., Maryland State Medical Journal 14: 56-61 (1965)).

Alternatively, stroke severity/outcome can be measured using the NIH stroke scale, which is available on the World Wide Web at ninds.nih.gov/doctors/NIH_Stroke_Scale_Booklet.pdf. This scale is based on the patient's ability to perform 11 sets of functions, including evaluations of the patient's levels of consciousness, motor function, sensation and linguistic function.

Ischemic stroke is more specifically defined as a type of stroke due to blockage of blood flow to the brain. The most common underlying disorder of such blockage is the formation of fatty deposits along the vessel wall. This condition is called atherosclerosis. These fatty deposits can lead to two kinds of obstruction. Cerebral thrombosis refers to thrombi (blood clots) formed at the site of vascular occlusion. "Cerebral embolism" generally refers to occlusion of cerebral arteries by various emboli carried by the bloodstream (e.g., intracardiac mural thrombi, atherosclerotic plaques, fat, tumor cells, fibrocartilage, or air). When collateral circulation fails to compensate, ischemic necrosis occurs in brain tissue within the arterial supply territory, resulting in focal neurological deficits. A second major cause of cerebral embolism is an irregular heartbeat known as atrial fibrillation. This disorder enables blood clots to form inside the heart, which may dislodge and travel to the brain. Other potential causes of ischemic stroke include hemorrhage, thrombosis, arterial or venous dissection, cardiac arrest, shock caused by any cause including hemorrhage, and iatrogenic causes such as direct surgical injury to cerebral vessels or vessels leading to the brain, or cardiac surgery. Ischemic stroke accounts for about 83% of all stroke cases.

In a fourth aspect, the present invention provides use of the pharmaceutically acceptable salt of the polypeptide of the first aspect or the pharmaceutical composition of the second aspect for treating, ameliorating or preventing stroke, Alzheimer's disease, spinal cord injury, aortic stenosis, or neonatal hypoxic-ischemic encephalopathy in a subject. Accordingly, the present invention also provides use of the pharmaceutically acceptable salt of the polypeptide of the first aspect or the pharmaceutical composition of the second aspect in the manufacture of a medicament for treating, ameliorating or preventing stroke, Alzheimer's disease, spinal cord injury, aortic stenosis, or neonatal hypoxic-ischemic encephalopathy in a subject.

Similarly, the present invention provides use of the pharmaceutically acceptable salt of the polypeptide of the first aspect or the pharmaceutical composition of the second aspect for treating, ameliorating or preventing ETBR-related diseases in a subject. Accordingly, the present invention also provides use of the pharmaceutically acceptable salt of the polypeptide of the first aspect or the pharmaceutical composition of the second aspect in the manufacture of a medicament for treating, ameliorating or preventing ETBR-related diseases in a subject.

In some embodiments, the ETBR-related diseases include one or more of neurological injury and secondary disorders resulting therefrom, neurodegenerative diseases, anxiety disorders, epileptic disorders, aortic stenosis, and neonatal hypoxic-ischemic encephalopathy.

In some embodiments, the neurological injury and secondary disorders resulting therefrom include stroke, spinal cord injury, ischemic or traumatic brain or spinal cord injury, and central nervous system (CNS) neuronal injury.

In some embodiments, the neurodegenerative diseases include Alzheimer's disease, amyotrophic lateral sclerosis (ALS), Parkinson's disease, and Huntington's disease;
In some embodiments, the stroke includes ischemic stroke, hemorrhagic stroke, and hemorrhagic stroke converted from ischemic stroke.

As used herein, "subject" refers to an animal comprising birds, reptiles, and mammals. In some embodiments, the subjects are mammals, including primates and non-primates such as humans, chimpanzees, cattle, horses, pigs, sheep, goats, canines, cats, and rodents such as rats and mice.

It is to be understood that the foregoing detailed description is only for those skilled in the art to understand the contents of the present application more clearly and is not intended to be limiting in any way. Various modifications and variations can be made to the described embodiments by those skilled in the art.

In the present application, the interpretation of the abbreviations or the full English names is as follows.

| Abbreviations | Full name |
|---|---|
| Boc | tert-butoxycarbonyl |
| DCM | dichloromethane |
| DIEA | diisopropylethylamine |
| DMF | N,N-dimethylformamide |
| MeOH | methanol |
| Fmoc | Fluorenylmethyloxycarbonyl |
| HBTU | O-benzotriazole-tetramethyl-uronium-hexafluorophosphate |
| OtBu | tert-butoxy(-O-C(CH₃)₃) |
| Dde | 1-(4,4-dimethyl-2,6-dioxocyclohexylidene)ethyl |
| Resin | resin |
| Trt | Triphenylmethyl |
| TFA | trifluoroacetic acid |
| TIS | triisopropylsilane |
| Suc | succinyl |
| Fum | fumaryl |
| AEEA | aminoethoxyethoxyacetic acid |
| C12 | lauroyl |

### Examples

The following examples are used to illustrate the present invention, but are not intended to limit the scope of the present invention. Modifications or substitutions of the methods, steps or conditions of the present invention shall fall within the scope of the present invention without departing from the spirit and scope of the present invention.

Unless otherwise specified, the reagents used in the examples are all conventional commercially available reagents, and the technical means used in the examples are all conventional means well known to those skilled in the art.

Unless otherwise specified, the main reagents and solvents used in the following examples are all commercially available, for example, from GL Biochem (Shanghai) Co., Ltd., or Sangon Biotech (Shanghai) Co., Ltd. Control IRL-1620 was synthesized and obtained according to the sequence and synthesis method described in Michihiro Takai, et al., BIOCHEMICAL AND BIOPHYSICAL RESEARCH COMMUNICATIONS, Vol. 184, No. 2, 1992, Pages 953-959.

### Example 1: Preparation of Polypeptide BX-229 and Salts Thereof

In this example, the polypeptide BX-229 (SEQ ID NO: 1, Fum-DEEAVYFAHK(-AEEA-γE-C12)DVIW) and its various salts were synthesized according to the following methods.

### 1) Synthesis of BX-229

0.50 g (0.25 mmol) of Fmoc-Trp(Boc)-Wang Resin was weighed and added into a reactor. 10 mL DCM was added for swelling for 10 min, then filtered by suction, and washed twice with DMF. 25% 4-methylpiperidine/DMF (by volume) was added and allowed to react for 30 min to remove the Fmoc group. After filtering by suction, the resin was washed four times with DMF and twice with DCM. A ninhydrin test was performed, and the resin solution was found to be blue. Fmoc-Ile-OH (1 mmol, 4 eq.) and HBTU (1 mmol, 4 eq.) were weighed and dissolved in DMF, and DIEA (2 mmol, 8 eq.) was added and mixed to uniformity. The mixture was added to the resin and allowed to react for 1 h under magnetic stirring at room temperature (25 °C ± 5 °C). After filtering by suction, it was washed sequentially with DMF, MeOH, and DCM, three times each. A ninhydrin test was performed, and the resin was found to be colorless and transparent, while the solution was found to be light yellow, indicating a complete reaction. 25% 4-methylpiperidine/DMF (by volume) was added to remove the Fmoc group. This cycle was repeated to sequentially complete the coupling of Val, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF was added to the peptide resin and allowed to react for 10 min for 3 times to remove the Dde on the side chain of Lys. AEEA, Glu-α-OtBu, and C12-OH were then condensed sequentially. The peptide resin was dried under suction, and 15 mL pre-cooled cleavage solution (TFA: TIS: H₂O = 95:2.5:2.5) was added. The reaction was allowed to proceed for 3 h at room temperature (25 °C ± 5 °C) with stirring. The reaction solutions were extracted by suction, and the resin was washed twice with a small amount of TFA and then extracted by suction. The reaction solutions were combined, and approximately 200 mL pre-cooled methyl tert-butyl ether was added. A white precipitate was formed. After centrifuging (5000 rpm for 5 min), the supernatant was discarded. Methyl tert-butyl ether was added again to the precipitate. After vortexing and centrifuging, the supernatant was discarded. The precipitate was placed in a vacuum dryer and dried for 12 h to obtain a crude product. The crude peptide was dissolved in approximately 1% aqueous ammonia, filtered, and the filtrate was purified with preparative liquid phases. Preparative column: C18-10-100, 30×250 mm. Flow rate: 25 mL/min. Phase A: 0.1% TFA/water, and Phase B: 0.1% TFA/90% acetonitrile/water. A gradient elution was performed to obtain a purified target compound. Fractions of the target compound with a purity >95% were combined, and acetonitrile was removed under reduced pressure at around 40 °C using a water pump and a rotary evaporator. The resulting concentrated solution was frozen into a solid via liquid nitrogen and lyophilized for 48 h in a freeze-dryer to obtain the target compound BX-229. The molecular weight was identified to be correct by mass spectrometry ([M-2]²⁻=1137.3).

### 2) Preparation of Sodium Salt of BX-229

The resulting BX-229 solid was dissolved in 0.1 N of aqueous sodium hydroxide solution (containing 10 equivalents of sodium hydroxide) and converted into a salt using preparative liquid phases. Preparative column: C18-10-100, 30×250 mm. Flow rate: 25 mL/min. Phase A: water, and Phase B: acetonitrile. After loading, it was rinsed with water for 15 min, and then the product was eluted from the preparative column by gradient elution. Fractions of the target compound with a purity >95% were combined, and acetonitrile was removed under reduced pressure at around 40 °C using a water pump and a rotary evaporator. The concentrated solution was frozen into a solid via liquid nitrogen and lyophilized for 48 h in a freeze-dryer to obtain the target compound, i.e., sodium salt of BX-229.

### 3) Preparation of Potassium Salt of BX-229

The BX-229 solid was dissolved in 0.1 N of aqueous potassium hydroxide solution (containing 10 equivalents of potassium hydroxide) and converted into a salt using preparative liquid phases. Preparative column: C18-10-100, 30×250 mm. Flow rate: 25 mL/min. Phase A: water, and Phase B: acetonitrile. After loading, it was rinsed with water for 15 min, and then the product was eluted from the preparative column by gradient elution. Fractions of the target compound with a purity >95% were combined, and acetonitrile was removed under reduced pressure at around 40 °C using a water pump and a rotary evaporator. The concentrated solution was frozen into a solid via liquid nitrogen and lyophilized for 48 h in a freeze-dryer to obtain the target compound, i.e., potassium salt of BX-229.

### 4) Preparation of Ammonium Salt of BX-229

The BX-229 solid was dissolved in 5% aqueous ammonia solution (pH about 9) and converted into a salt using preparative liquid phases. Preparative column: C18-10-100, 30×250 mm. Flow rate: 25 mL/min. Phase A: water, and Phase B: acetonitrile. After loading, it was rinsed with water for 15 min, and then the product was eluted from the preparative column by gradient elution. Fractions of the target compound with a purity >95% were combined, and acetonitrile was removed under reduced pressure at around 40 °C using a water pump and a rotary evaporator. The concentrated solution was frozen into a solid via liquid nitrogen and lyophilized for 48 h in a freeze-dryer to obtain the target compound, i.e., ammonium salt of BX-229.

### Example 2: EC50 Determination for activity of BX-229-Na toward ETB receptor derived from various species

In this example, the agonist activity of BX-229-Na prepared in Example 1 toward ETB receptors derived from various species was tested and compared with that of IRL-1620.

293T-17 cells were seeded at a density of 650,000 cells per well in a 6-well plate and cultured overnight in an incubator at 37 °C and 5% CO₂. Then, the cells were transfected with an expression vector, PCDNA3.1-hETB (human ETB sequence, NCBI accession number: NM_000115.5) containing human ETB receptor gene, at a plasmid concentration of 2.5 µg per well. The cells were further cultured for 48 h. When green fluorescence expression was observed under a fluorescence microscope, the cells were then digested with trypsin and counted. The activity of BX-229-Na toward the human ETB receptor was determined using the IP-One Gq kit from Cisbio Bioassays. The cells were suspended in Stim Buffer provided in the kit and the cell suspension was added to a 96-well detection plate at 7 µL cell suspension (containing 30,000 cells) per well. BX-229-Na prepared in Example 1 and IRL-1620 were respectively diluted with Stim B to generate 11 concentration gradients, each concentration gradient was further diluted with Stim B to a 2× concentration, then 7 µL was added to the test cells. Meanwhile, 7 µL PBS was added to cells in negative control wells. The plate was sealed and incubated at 37 °C for 1 h in a cell culture incubator. Detection test wells, positive control wells (human endothelin ET1 (SEQ ID NO: 2, CSCSSLMDKECVYFCHLDIIW (C1-C15,C3-C11))), and PBS control wells were each added with 3 µL IP1 d2 working solution and 3 µL IP1 Tb cryptotate Antibody working solution. The plate was then sealed and incubated in dark at room temperature (25 °C ± 5 °C) for 1 h. The plate seal was removed, and the absorbance values at 665 nm and 620 nm were measured using an ID5 microplate reader. The ratio of the acceptor emission signal to the donor emission signal, i.e., the value of (signal at 665 nm/signal at 620 nm) ×10⁴, was calculated for each well. The average value of the signal ratio (665 nm/ 620 nm) determined for the test sample at various concentrations was defined as F. The activation rate at each concentration of the test polypeptides relative to human endothelin ET-1 (positive control) was first calculated. A dot plot was generated with activation rate on the vertical axis and log polypeptide concentrations on the horizontal axis. A curve was fitted using the four-parameter logistic model in Prism software, and the EC50 value of the polypeptide toward human ETB receptor was calculated.

The procedure for EC50 determination of activity toward ETB receptor derived from canine, rat and mouse was as described above, except that the transfected plasmids were the expression vector pcDNA3.1-dETB containing the canine ETB receptor gene (canine ETBR sequence, NCBI accession number: NM_001010943.2), pcDNA3.1-rETB containing the rat ETB receptor gene (rat ETB receptor sequence, NCBI accession number: X57764.1), and pcDNA3.1-mETB containing the mouse ETB receptor gene (mouse ETB sequence, NCBI accession number: NM_001276296).

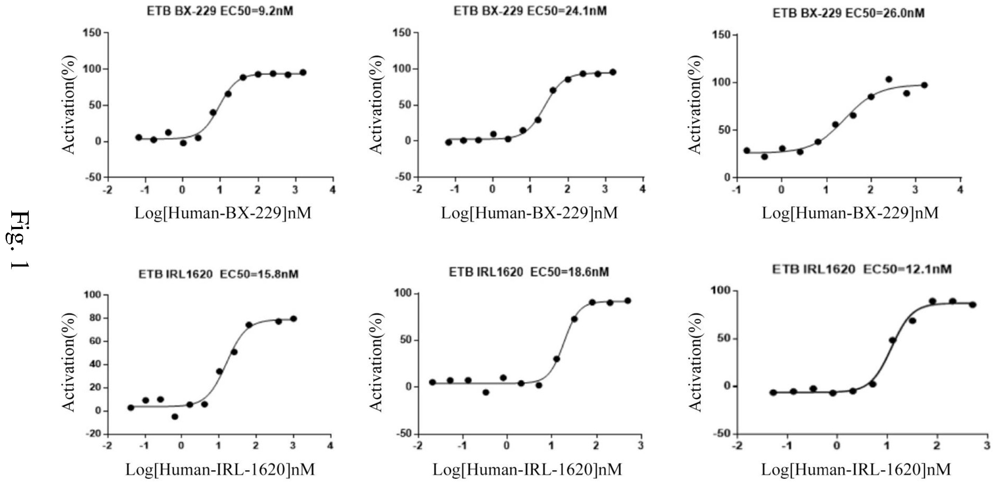

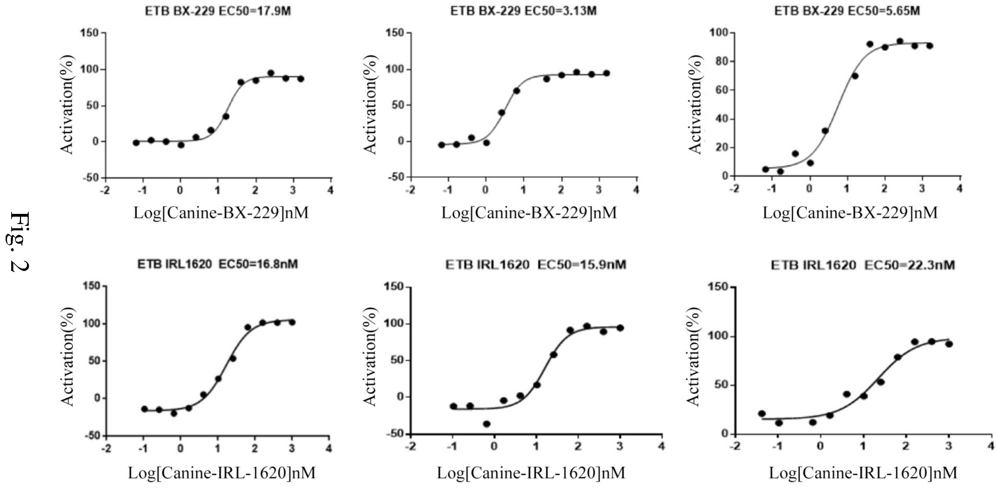

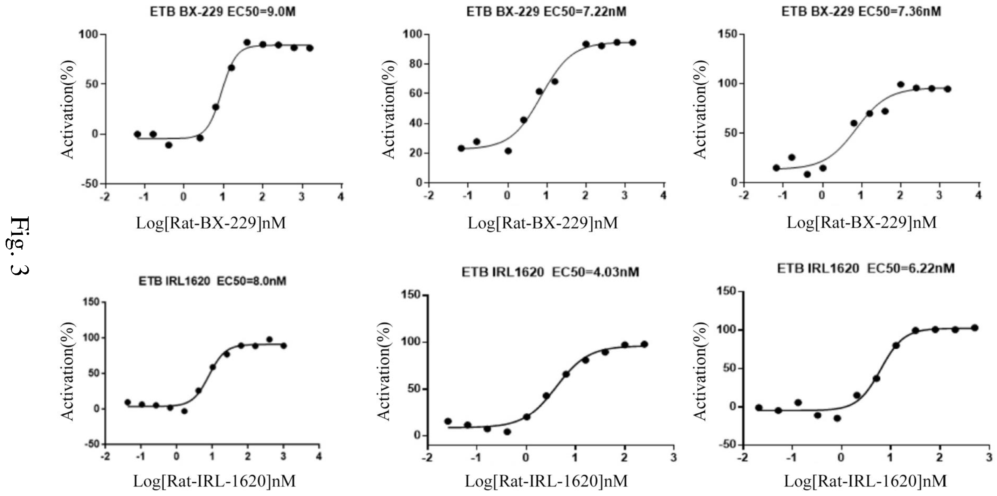

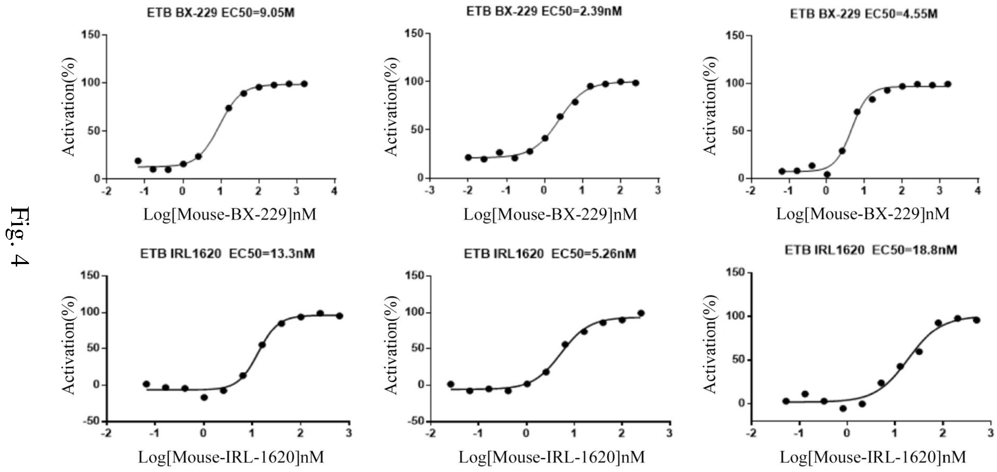

To obtain more reliable data, parallel comparison assays were carried out in the same experimental batch, and all assays were replicated multiple times. The three datasets exhibiting optimal curve fitting was selected and averaged, and the calculated mean values were defined as the final activity results, as shown in Table 1 and Figures 1 to 4.

**Table 1: Agonist Activity of BX-229-Na and IRL-1620 toward ETB Receptors (EC50, nM)**

| | Human | Canine | Rat | Mouse |
|---|---|---|---|---|
| IRL-1620 | 15.50±3.26 | 18.33±3.46 | 6.08±1.99 | 12.45±6.81 |
| BX-229-Na | 19.77±9.20 | 8.89±7.90 | 7.86±0.99 | 5.33±3.40 |

### Example 3: Activity of BX-229-Na toward Human ETA Receptor

In this example, the activity of BX-229-Na prepared in Example 1 toward the human ETA receptor was tested.

293T-17 cells were seeded at a density of 650,000 cells per well in a 6-well plate and cultured overnight in an incubator at 37°C and 5% CO₂.Then, the cells were transfected with an expression vector, pcDNA3.1-hETA (hETA sequence, NCBI Accession Number: L06622.1) containing the human ETA receptor gene, at a plasmid concentration of 2.5 µg per well. The cells were further cultured for 48 h. When green fluorescence expression was observed under a fluorescence microscope, the cells were then digested with trypsin and counted. The activity of BX-229-Na toward the human ETA receptor was determined using the IP-One Gq kit from Cisbio Bioassays. The cells were suspended in Stim Buffer provided in the kit and the cell suspension was added to a 96-well detection plate at 7 µL cell suspension (containing 30,000 cells) per well. The polypeptide prepared in Example 1 (i.e., BX-229-Na) and polypeptide human endothelin ET1 (SEQ ID NO.: 2) as positive control were respectively diluted with Stim B to generate a 2× concentration, then 7 µL was added to the test cells. Meanwhile, 7 µL PBS was added to cells in negative control wells. The plate was sealed and incubated at 37°C for 1 h in a cell culture incubator. Detection test wells, positive control wells, and PBS control wells were each added with 3 µL IP1 d2 working solution and 3 µL IP1 Tb cryptotate Antibody working solution. The plate was sealed and incubated in dark at room temperature (25 °C ± 5 °C) for 1 h. The plate seal was removed, and the absorbance values at 665 nm and 620 nm were measured using an ID5 microplate reader. The ratio of the acceptor emission signal to the donor emission signal, i.e., the value of (signal at 665 nm/signal at 620 nm) ×10⁴, was calculated for each well. With F3 representing the average ratio of signal at 665 nm/signal at 620 nm determined for the test sample at a specific concentration, F5 representing the average ratio of signal at 665 nm/signal at 620 nm determined for the positive control human endothelin ET1 at the same concentration, and T3 representing the average ratio of signal at 665 nm/signal at 620 nm determined for the PBS negative control, the percentage of activity of the test polypeptide sample relative to positive control sample ET1 = (F3-T3)×100/(F5-T3). Results were shown in Table 2, indicating that BX-229-Na was a selective ETB receptor agonist.

**Table 2: Activity of BX-229-Na toward Human ETA Receptor**

| | hETAR agonist activity | MW |
|---|---|---|
| BX-229-Na | 0.68%@100µM | 2276 |

### Example 4: Determination of Half-life of Polypeptide BX-229

In this example, the half-life of BX-229 prepared in Example 1 was tested and compared with that of IRL-1620.

Administration and process for plasma samples were as follows. Three C57BL/6 mice were injected with BX-229 and the control IRL-1620 respectively at a concentration of 60 µg/mL, with each mouse receiving an injection volume of 200 µL. Blood samples were collected at 1 min, 5 min, 15 min, and 30 min post-administration. Immediately following blood collection, protease inhibitor was added to each sample, and the samples were centrifuged at 3,200 rpm and 4 °C for 10 min. The supernatant plasma was taken, and then two-fold volume of acetonitrile was added for protein precipitation. The mixture was further centrifugated at 10,000 rpm for 5 min. After being filtered through a 0.22 µm filter membrane, the filtrate was analyzed via liquid chromatography-tandem mass spectrometry. Plasma drug concentrations were calculated from the integral areas of the control drug and the test drug. The average of the three replicate measurements at each time point was calculated for each test drug. Meanwhile, a blank mouse plasma sample was processed in parallel. Results were shown in Table 3.

**Table 3: Half-life Data of BX-229 and IRL-1620**

| Compound | Dose, i.v. | Half-life (min) | AUC_{last 0-30 min} (ng.h/mL) |
|---|---|---|---|
| IRL-1620 | 0.48 mpk | <2.6 | 130 |
| BX-229 | 1 mpk | 77 | 3393 |

Conditions for the liquid chromatography-mass spectrometry were as follows.
Instrument model: Agilent InfinityLab LC/MSD 1260-G6125C
Chromatographic column: Poroshell 120 SB-C18 4.6*100 mm, 2.7 µm
Mobile phase A: 0.1% formic acid-ultrapure water
Mobile phase B: 0.1% formic acid-acetonitrile

### Gradient settings:

| Time (min) | Phase A | Phase B | Flow rate |
|---|---|---|---|
| 0 | 90% | 10% | 0.4mL/min |
| 18 | 0 | 100% | 0.4mL/min |
| 20 | 0 | 100% | 0.4mL/min |

Column temperature: 45°C; Sample volume loaded: 20 µL;
Ion source: ESI; Dryer temperature: 350°C; Dryer flow rate: 12 L/min; Nebulizing pressure: 45 psi; Capillary voltage: 4000 V.
Scan type: SIM; Fragmentor voltage: 135 V; Scan/dwell time: 200 ms.

### Example 5: Preliminary Study on Acute Toxicity of Intravenous Administration of Polypeptide BX-229

In this example, a preliminary study on acute toxicity of intravenous administration of BX-229 prepared in Example 1 was carried out and compared with that of IRL-1620.

In the intravenous acute toxicity test, male SD rats (body weight: 200-230 g) were given tail vein injections of BX-229 or IRL-1620 at sequentially descending doses ranging from 120 µg/kg to 30 µg/kg. The animals were observed for 24 h post-administration. If a certain dose resulted in high rat mortality, the injection dose was lowered for subsequent observation. Toxic response and mortality of rats were preliminarily monitored, and the time of death and no-mortality doses levels were recorded. The level of acute toxicity of the polypeptides was preliminarily evaluated based on the no-mortality doses. Results were shown in Table 4. The acute toxicity study showed that the maximum tolerated doses of IRL1620 and BX229 in rats were 30 µg/kg and 60 µg/kg, respectively. The maximum tolerated dose of BX229 in SD rats following intravenous injection was markedly higher than that of IRL1620.

**Table 4: Acute Toxicity Test Results of Intravenous Administration of BX-229 and IRL-1620**

| Compound | Animal No. | Intravenous Administratio n Dose | Observations of Animal Status |
|---|---|---|---|
| IRL-1620 | 1 | 120 µg/kg | Tachypnea and cyanosis observed at 5 min post-administration, and death occurred 4 min later |
| | 2 | 60 µg/kg | Tachypnea observed at 3 min post-administration, and death occurred 5 min later |
| | 3 | 30 µg/kg | Tachypnea observed at 13 min post-administration, hypoactivity lasted for 17 min, resolution at 1.5 h later |
| | 4 | 30 µg/kg | Tachypnea observed at 15 min post-administration, hypoactivity lasted for 10 min, resolution at 1.5 h later |
| | 5 | 30µg/kg | Motionless at 25 min post-administration, hypoactivity lasted for 65 min, dead the following morning (death presumed to have occurred overnight) |
| BX-229 | 1 | 120 µg/kg | Death occurred at 26 min post-administration, nasal white discharge and diarrhea noted |
| | 2 | 120 µg/kg | Death occurred at 26 min post-administration; nasal white discharge and diarrhea noted |
| | 3 | 90 µg/kg | Death occurred at 1 h post-administration |
| | 4 | 90 µg/kg | Tachypnea observed at 25 min post-administration, lasted for 25 min with gradual resolution; hypoactivity observed |
| | 5 | 90 µg/kg | Tachypnea and cyanosis observed at 1 h post-administration, lasted for 30 min with gradual resolution |
| | 6 | 90 µg/kg | Tachypnea and diarrhea observed at 45 min post-administration, lasted for 25 min with gradual resolution |
| | 7 | 90 µg/kg | Tachypnea observed at 44 min post-administration, followed by convulsions and death 8 min later |
| | 8 | 90 µg/kg | Tachypnea observed at 43 min post-administration, lasted for 30 min with gradual resolution |
| | 9 | 60 µg/kg | No obvious reaction observed within 1 h post-administration, tachypnea developed at 1 h, lasted for 10 min with gradual resolution |
| | 10 | 60 µg/kg | No obvious reaction observed within 1 h post-administration, tachypnea developed at 1 h, lasted for 10 min with gradual resolution |

### Example 6: Preliminary Study on Acute Toxicity of Subcutaneous Administration of Polypeptide BX-229

In this example, a preliminary study on acute toxicity of subcutaneous administration of BX-229 prepared in Example 1 was carried out and compared with that of IRL-1620.

In subcutaneous acute toxicity test, male SD rats (body weight: 200-230 g) were subcutaneously administered BX-229 or IRL-1620 respectively, with doses ranging from 60 µg/kg to 960 µg/kg. The animals were observed for 48 h post-administration. Toxic response and mortality of the rats were preliminarily monitored, and the time of death and no-mortality doses levels were recorded. The level of acute toxicity of the polypeptides was preliminarily evaluated based on the no-mortality doses. Results were shown in Table 5. It can be seen from the table that the acute toxicity of subcutaneous administration of BX-229 was much lower than that of IRL-1620.

**Table 5: Acute Toxicity Test Results of Subcutaneous Administration of BX-229 and IRL-1620**

| Compound | Animal No. | Subcutaneous Administration Dose | Observations of Animal Status |
|---|---|---|---|
| IRL-1620 | 1 | 60 µg/kg | Tachypnea observed at 25 min post-administration, lasted for 15 min with resolution |
| | 2 | 60 µg/kg | Tachypnea observed at 26 min post-administration, lasted for 16 min with resolution |
| | 3 | 60 µg/kg | Tachypnea observed at 25 min post-administration, lasted for 15 min with resolution |
| | 4 | 60 µg/kg | Tachypnea observed at 30 min post-administration, lasted for 17 min with resolution |
| | 5 | 60 µg/kg | Tachypnea observed at 25 min post-administration, lasted for 16 min with resolution |
| | 6 | 120 µg/kg | Tachypnea observed at 26 min post-administration, and death occurred 23 min later |
| | 7 | 120 µg/kg | Tachypnea observed at 26 min post-administration, lasted for 24 min with resolution |
| | 8 | 120 µg/kg | Tachypnea observed at 26 min post-administration, and death occurred 26 min later |
| | 9 | 120 µg/kg | Tachypnea observed at 28 min post-administration, lasted 22 min with resolution |
| | 10 | 120 µg/kg | Tachypnea observed at 27 min post-administration, lasted 23 min with resolution |
| BX-229 | 1 | 120 µg/kg | No obvious reaction observed for 24 h post-administration |
| | 2 | 120 µg/kg | No obvious reaction observed for 24 h post-administration |
| | 3 | 240 µg/kg | No obvious abnormal reaction observed for 48 h post-administration |
| | 4 | 240 µg/kg | No obvious abnormal reaction observed for 48 h post-administration |
| | 5 | 480 µg/kg | No obvious abnormal reaction observed for 48 h post-administration |
| | 6 | 480 µg/kg | No obvious abnormal reaction observed for 48 h post-administration |
| | 7 | 960 µg/kg | No obvious abnormal reaction observed for 2 h post-administration, and death occurred after 10 min |
| | 8 | 960 µg/kg | No obvious abnormal reaction observed for 48 h post-administration |
| | 9 | 960 µg/kg | No obvious abnormal reaction observed for 48 h post-administration |
| | 10 | 960 µg/kg | No obvious abnormal reaction observed for 48 h post-administration |
| | 11 | 960 µg/kg | No obvious abnormal reaction observed for 48 h post-administration |

### Example 7: Effect of Intravenous Administration of BX-229 on Blood Pressure in SD Rats

In this example, the effect of intravenous administration of BX-229 prepared in Example 1 on blood pressure in SD rats was tested and compared with that of IRL-1620.

Male SD rats were anesthetized with isoflurane, and their neck fur were shaved. A midline incision was made on the neck to expose the trachea. A ventilator and anesthesia machine for small animals were activated, with concentration of an anesthetic gas set to 1.2, and the anesthesia machine outlet connected to the ventilator inlet. The ventilator tidal volume was set to 2 mL, respiratory rate to 90 breaths/min, and inspiratory-to-expiratory ratio to 1:2. The ventilator outlet was connected to a plastic catheter, which was cannulated into the rat's airway.

The hardware and supporting software of the biological signal data acquisition and analysis system were activated. One port of the acquisition board was connected to the plastic catheter, and the other port was connected to a connector valve attached to a 1-mL syringe prefilled with heparinized saline. Heparinized saline was injected into the data acquisition board to expel the air in the entire space until the heparinized saline flowed out from the other end of the plastic catheter. The right common carotid artery of the rat was isolated. A dead knot was made at the distal end and a slipknot at the proximal end of a right common carotid artery. A 1/2-sized incision was made in the artery between the knot with microscissors, and the plastic catheter with heparinized saline was cannulated into the incision and fixed to prevent back-and-forth movement. The initial value of the blood pressure was set to zero with the zero-adjustment valve of the biological signal data acquisition and analysis system. The slipknot at the proximal end of the common carotid artery was released, and real-time blood pressure data of the animal were displayed on the software. After the animal's blood pressure was stabilized, the drug was administered via tail vein, and changes in the animal's blood pressure were observed in real-time. The dose of IRL-1620 was 5 µg/kg, and the dose of BX-229 was 6.6 µg/kg.

Results were shown in Table 6. It can be seen that 5 µg/kg IRL-1620 and 6.6 µg/kg BX-229 had certain effects on the rats' blood pressure, causing a decrease in blood pressure. After administration of 5 µg/kg IRL-1620, the rats' blood pressure dropped rapidly and reached the lowest level within 2 min, with a maximum blood pressure reduction of 16.1% ± 2.4% relative to baseline. At 6.6 µg/kg, BX-229 had a relatively mild effect on the rats' blood pressure, causing a slow decrease in blood pressure to the lowest blood pressure at 20-35 min, with the a maximum blood pressure reduction of 17.0% ± 2.0% relative to baseline.

**Table 6: Effects of Intravenous Administration of BX-229 and IRL-1620 on Blood Pressure in SD Rats**

| Compound (Administration Concentration) | IRL-1620(5µg/kg) | BX-229(6.6µg/kg) |
|---|---|---|
| Maximum percentage of blood pressure reduction relative to baseline (n = 3) | 16.1%±2.4% | 17.0%±2.0% |
| Time from baseline to the lowest blood pressure | Less than 2 min | 20-30 min |

### Example 8: Comparative Experiment on Stability of Different Salt Forms of BX-229

### 1. Preparation of samples

Solution preparation: 1 mg each raw material was weighed into a 1.5 mL centrifuge tube, and 1 mL normal saline was added to dissolve the solid. 3 tubes of solution were prepared for each raw material, and 1 tube was placed at 40°C, 25°C and 5°C, respectively.

Powder: 1 mg each raw material was weighed into a 1.5 mL centrifuge tube. 6 tubes of powder were prepared for each raw material, and 3 tubes were placed at 40°C and 25°C, respectively.
2. Detection method: HPLC method
Column: Poroshell 120 SB-C18, 2.7 µm, 4.6×100 mm
Gradient: 10-100% B, 0-10 min, flow rate: 2 mL/min
Column temperature: 35 °C
Injection volume: 5 µL
Mobile phase A: 0.05%TFA, 2% acetonitrile, 98% ultrapure water
Mobile phase B: 0.05%TFA, 10% ultrapure water, 90% acetonitrile
3. Detection results:
The purity (%) of samples of different salt forms after standing under different conditions was determined by HPLC, as shown in Table 7.

**Table 7: Purity (%) of Samples of Different Salt Forms of BX-229 After Standing Under Different Conditions**

| | Sodium salt powder | Potassium salt powder | Ammonium salt powder | Sodium salt solution | Potassium salt solution | Ammonium salt solution |
|---|---|---|---|---|---|---|
| Day 0 | 99.6 | 99.6 | 97.23 | 99.6 | 99.6 | 97.23 |
| 40°C, 1 week | 99.6 | 99.6 | 97.92 | 99.6 | 99.6 | 94.26 |
| 40°C, 2 weeks | 99.6 | 99.6 | 97.96 | 98.74 | 99.67 | 94.69 |
| 40°C, 4 weeks | 99.4 | 99.42 | 96.51 | 97.53 | 97.86 | 79.51 |
| 25°C, 1 month | 99.76 | 99.63 | 97.81 | 99.64 | 98.42 | 88.51 |

It can be seen that after standing at 40°C for 1 or 2 weeks, the sodium and potassium salt powders were stable and the ammonium salt powder was not very stable; the sodium and potassium salt solutions were stable and the ammonium salt solution was unstable. After standing at 40°C for 4 weeks, the sodium and potassium salt powders remained stable and the ammonium salt powder was not very stable; the sodium and potassium salt solutions were not very stable, and the ammonium salt solution was very unstable. After standing at 25°C for 1 month, the sodium and potassium salt powders were stable and the ammonium salt powder was not very stable; and the sodium salt solution was stable.

### Example 9: Efficacy of BX229 Sodium Salt Subcutaneously Administered Three Times per Week on Mouse Model of tMCAO

In this example, the efficacy of the BX-229 sodium salt (BX-229-Na) prepared in Example 1 on a mouse tMCAO model was tested.

Fifty-one male C57BL/6J mice, aged 10-12 weeks (body weight 25-30 g), were housed for at least 2 days prior to the experiment to for acclimatization (SPF Biotech). A mouse tMCAO model was established. The mice were anesthetized with isoflurane. Core body temperature was maintained at 37 ± 0.5°C using a thermostatic blanket. A midline skin incision was made in skull, the skin was retracted outward, and a bendable microneedle tip was fixed on the surface of the left parietal bone. A midline cervical incision was made to isolate the right common carotid artery (CCA), external carotid artery (ECA) and internal carotid artery (ICA). According to the method described by Longa et al., 1989, a silicone rubber-coated suture (RWD Life Science, suture end diameter: 0.22 mm-0.23 mm, MSMC23B120PK50) was inserted via an external carotid artery incision and advanced slowly along the internal carotid artery toward the origin of the middle cerebral artery. To ensure persistent middle cerebral artery occlusion, local cerebral blood flow of all modeled mice was monitored using a laser speckle flowmeter (blood flow was reduced by 40-60% after stabilization). The suture was withdrawn 45 min after insertion, and the incision was sutured. Subsequently, 1 mL warm saline (37°C) was injected subcutaneously, and mice were placed on a heating pad until recovery. To alleviate pain, mice were injected subcutaneously with tilidine after anesthesia recovery. The modeling day was defined as Day 1 (D1), the next day as D2, and so on.

Successfully modeled C57BL/6J tMCAO mice were randomly assigned into three groups: normal saline group, 30 µg/kg BX-229-Na group, and 100 µg/kg BX-229-Na group. All mice received subcutaneous neck administration at 4.5 h after occlusion on D1, followed by additional administration at the same site and time point on D3 and D6. The grouping and dosing regimen were shown in Table 8. The sham group included 6 mice, which received 2 mL/kg normal saline via subcutaneous neck injection. Garcia JH scoring was made on D7 and D14 post-administration. Upon completion of the scoring on D14, all mice were euthanized, and infarcted brain tissues were collected for total protein extraction. Changes in protein markers VEGF-A and BDNF in each group were determined by ELISA.

**Table 8: Experimental Grouping and Dosing Regimen**

| Group | Number | t-MCAO Establishment | CPD | Dose (µg/kg) | Volume (mL/kg) | Administration Frequency |
|---|---|---|---|---|---|---|
| 1 | 15 | t-MCAO | Normal saline | - | 2 | D1: two-point subcutaneous neck injection administered at 4.5 h after tMCAO occlusion |
| 2 | 15 | t-MCAO | BX-229 | 30 | 2 | |
| 3 | 15 | t-MCAO | BX-229 | 100 | 2 | |
| 4 | 6 | Sham group | Normal saline | - | 2 | |
| | | | | | | D3 and D6: once administration at the identical time and site identical time and site |

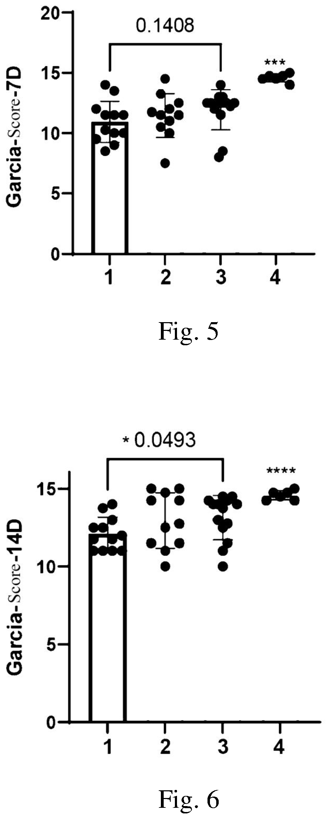

Results showed that according to Garcia JH-15 scoring on D7, there was a significant difference in D7 scores between the sham group and the normal saline control group. Both the BX-229-30 µg/kg and BX-229-100 µg/kg groups had higher scores than the normal saline group, showing a certain dose-dependent trend. The total Garcia JH-15 score of the BX-229-100 µg/kg group was 1 point higher than that of the normal saline group. According to Garcia JH-15 scoring on D14, there was a significant difference in D14 scores between the sham group and normal saline control group. Similarly, the BX-229-30 µg/kg and BX-229-100 µg/kg groups had higher scores than that of the normal saline group, showing a dose-dependent relationship, and the BX-229-100µg/Kg group had significantly higher Garcia JH-15 scores than the normal saline group (P < 0.05). See Figures 5 and 6.

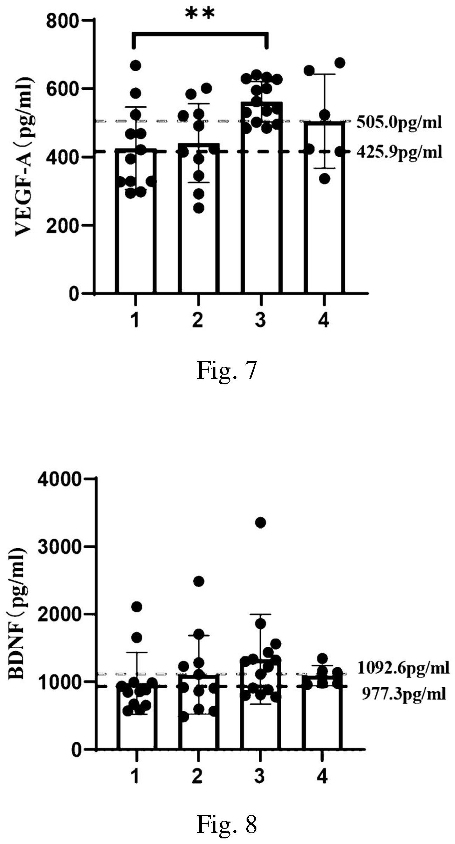

Results showed that the protein expression levels of VEGF-A and BDNF in the BX-229Na-30 µg/kg three-dose group and BX-229Na-100 µg/kg three-dose group were higher than those in the normal saline group, and the protein expression level of VEGF-A in the BX-229Na-100 µg/Kg three-dose group was higher than that in the sham group, with a significant difference compared with the normal saline group. Besides, BDNF protein expression in the BX-229Na-100 µg/Kg three-dose group was higher than that in the sham group. Results were shown in Figures 7 and 8, as well as Tables 9 and 10.

**Table 9: Changes in VEGF-A protein expression**

| Group | VEGF-A pg/ml |
|---|---|
| 1 | 426±115 |
| 2 | 441±102 |
| 3 | 562±56 |
| 4 | 505±122 |

**Table 10: Changes in BDNF protein expression**

| Group | BDNF pg/ml |
|---|---|
| 1 | 977±459 |
| 2 | 1103±582 |
| 3 | 1334±665 |
| 4 | 1093±151 |

### Example 10: Efficacy of Subcutaneous Administration of BX-229 Sodium Salt and Intravenous Administration of IRL-1620 in mouse tMCAO model

In this example, the efficacy of subcutaneous administration of the BX-229 sodium salt (BX-229-Na) prepared in Example 1 in mouse tMCAO model was tested and compared with that of intravenous administration of IRL-1620. The administration route and dose of IRL-1620 were determined via animal equivalent conversion based on clinical administration data. The modeling procedure for the mouse tMCAO model was referred to in Example 9.

After surgery, successfully modeled tMCAO mice were randomly assigned into a model group and various treatment groups. Detailed grouping was as follows: (1) 2.7 µg/kg IRL-1620 group: IRL-1620 was intravenously administered at 2.7 µg/kg three times daily on D1, D3, and D6 post-modeling; on D1, the first administration was performed at 2 h post-modeling, followed by subsequent administration every 2 h; the dosing schedule on D3 and D6 was the same as that on D1; (2) 30 µg/kg BX-229-Na group: BX-229 was subcutaneously administered at 30 µg/kg once daily for 7 consecutive days from D1 to D7; the administration time was at 4.5 h post-modeling on D1 and at the same time on D2 to D7; (3) model (normal saline) group; and (4) sham group: mice in the sham group received identical surgical procedures without vascular ligation and suture insertion. The model group and the sham group were given an equal volume of normal saline vehicle.

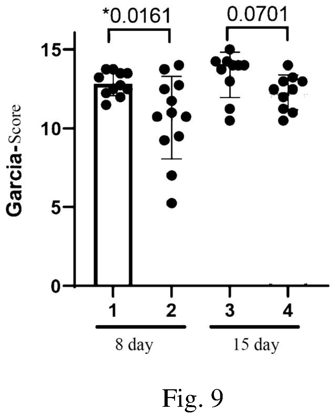

Results showed that 30 µg/kg BX-229-Na group (subcutaneous administration for 7 consecutive days from D1 to D7) obtained higher neurobehavioral scores than the 3 × 2.7 µg/kg IRL-1620 intravenous group (intravenous administration on D1, D3, and D6) on D7. Compared with the IRL-1620 group, mice in the BX-229-Na group exhibited greatly improved neurological function and significantly restored neurobehavioral scores, as detailed in Figure 9. It can be seen that subcutaneous BX-229-Na had a lower administration frequency, greater operability, and better patient compliance relative to intravenous IRL-1620 group.

The polypeptides and use thereof provided by the present invention are described in detail above. The principles and implementations of the present invention have been elucidated with reference to specific examples, the description of which is merely intended to assist in understanding the method of the invention and its core idea. It should be noted that those skilled in the art, without departing from the principles of the invention, may make several variations and modifications of the invention, which also fall within the scope of the claims.

## Claims

1. A pharmaceutically acceptable salt of a polypeptide, wherein the polypeptide comprises the amino acid sequence Fum-DEEAVYFAHK(-AEEA-γE-C12)DVIW(SEQ ID NO: 1) or a functional variant thereof, and the pharmaceutically acceptable salt is selected from the group consisting of a sodium salt, a potassium salt, an ammonium salt, trifluoroacetate, acetate, hydrochloride, sulfate, and phosphate.

2. The pharmaceutically acceptable salt of the polypeptide according to claim 1, wherein the pharmaceutically acceptable salt is selected from the group consisting of a sodium salt, a potassium salt and an ammonium salt, preferably a sodium salt.

3. The pharmaceutically acceptable salt of the polypeptide according to claim 1 or 2, wherein the functional variant of the polypeptide comprises substitutions between D and E, among V, L and I, among Y, F and W, among H, K and R in SEQ ID NO: 1, or any combination thereof, and has the same or similar Endothelin B receptor (ETBR) agonistic activity as the polypeptide of SEQ ID NO: 1.

4. A method of preparing the pharmaceutically acceptable salt of the polypeptide of any one of claims 1 to 3, comprising the steps of:
obtaining a peptide resin of the polypeptide via solid-phase polypeptide synthesis;
cleaving and purifying to obtain the polypeptide; and
preparing the salt from the polypeptide.

5. A pharmaceutical composition comprising the pharmaceutically acceptable salt of the polypeptide of any one of claims 1 to 3, and a pharmaceutically acceptable carrier, excipient and/or diluent.

6. The pharmaceutical composition according to claim 5, wherein the pharmaceutical composition is a pre-lyophilized formulation, or a lyophilized formulation, or a reconstituted formulation obtained by reconstituting the lyophilized formulation with an aqueous solution.

7. The pharmaceutical composition according to claim 6, wherein the pharmaceutical composition comprises sodium chloride; and/or optionally, the pharmaceutical composition comprises trehalose and/or cyclodextrin.

8. Use of the pharmaceutically acceptable salt of the polypeptide according to any one of claims 1 to 3, or the pharmaceutical composition according to any one of claims 5 to 7 in the manufacture of a medicament for the treatment of ETBR-related diseases;
preferably, the ETBR-related diseases comprise one or more selected from the group consisting of neurological injury and secondary disorders resulting therefrom, neurodegenerative diseases, anxiety disorders, epileptic disorders, aortic stenosis, and neonatal hypoxic-ischemic encephalopathy;
more preferably, the neurological injury and secondary disorders resulting therefrom are selected from the group consisting of stroke, spinal cord injury, ischemic or traumatic brain or spinal cord injury, and central nervous system (CNS) neuronal injury; or the neurodegenerative diseases are selected from the group consisting of Alzheimer's disease, amyotrophic lateral sclerosis (ALS), Parkinson's disease, and Huntington's disease;
more preferably, the stroke is selected from the group consisting of ischemic stroke, hemorrhagic stroke, and hemorrhagic stroke converted from ischemic stroke.

9. A medicament comprising the pharmaceutically acceptable salt of the polypeptide according to any one of claims 1 to 3, or the pharmaceutical composition according to any one of claims 5 to 7, a pharmaceutically acceptable additive or auxiliary, and optional any other active ingredient.

10. A method for treating ETBR-related diseases, comprising administering to a subject the pharmaceutically acceptable salt of the polypeptide of any one of claims 1 to 3, the pharmaceutical composition of any one of claims 5 to 7, or the medicament of claim 9;
preferably, the ETBR-related diseases comprise one or more selected from the group consisting of neurological injury and secondary disorders resulting therefrom, neurodegenerative diseases, anxiety disorders, epileptic disorders, aortic stenosis, and neonatal hypoxic-ischemic encephalopathy;
more preferably, the neurological injury and secondary disorders resulting therefrom are selected from the group consisting of stroke, spinal cord injury, ischemic or traumatic brain or spinal cord injury, and central nervous system (CNS) neuronal injury;
more preferably, the neurodegenerative diseases are selected from the group consisting of Alzheimer's disease, amyotrophic lateral sclerosis (ALS), Parkinson's disease, and Huntington's disease;
more preferably, the stroke is selected from the group consisting of ischemic stroke, hemorrhagic stroke, and hemorrhagic stroke converted from ischemic stroke.
